# EUROPEAN PATENT SPECIFICATION

(11) **EP 0 814 772 B1**
(45) Date of publication and mention of the grant of the patent: **18.07.2001**
(21) Application number: 96908088.6
(22) Date of filing: 16.03.1996
(51) Int. Cl.: A61K 9/00

(54) **PECTIN PHARMACEUTICAL COMPOSITIONS**
PHARMAZEUTISCHE ZUSAMMENSETZUNGEN MIT PEKTIN
COMPOSITIONS PHARMACEUTIQUES A BASE DE PECTINE

(30) Priority: 17.03.1995 GB 9505369; 05.04.1995 GB 9507038
(43) Date of publication of application: 07.01.1998
(73) Proprietor: The Boots Company PLC, Nottingham, Nottinghamshire NG2 3AA (GB)
(72) Inventor: COX, Gillian, Nottingham NG2 3AA (GB)
(74) Representative: Thacker, Michael Anthony
(86) International application number: EP9601204
(87) International publication number: WO9629054

(56) References cited:
- EP-A- 0 286 085
- GB-A- 2 283 171
- JOURNAL OF CONTROLLED RELEASE, vol. 30, no. 3, 1994, pages 225-232, XP000453419 MARIANNE ASHFORD, ET AL.: "Studies on pectin formulations for colonic drug delivery"

## Description

The present invention relates to pharmaceutical compositions used to treat disorders of the digestive system. In particular, the invention relates to solid preparations for use in the prevention of gastric reflux.

Gastric reflux is the passage of small amounts of highly acidic gastric juice and bile acids from the stomach into the lower part of the oesophagus. This causes the pain of heartburn and can inflame or damage the oesophagus. Gastric reflux may be symptomatic of a variety of disorders such as ulcers or biliary conditions.

Preparations which are known to treat gastric reflux comprise a substance which when exposed to the acid contents of the stomach forms a gel which floats on the surface of the stomach contents as a raft due to a lower bulk density. The lower bulk density is achieved by entrapment of gas bubbles within the gel structure. The gas is generated by a material which produces gas in the acidic conditions of the stomach as the gel is being formed, for example a carbonate or bicarbonate which produces carbon dioxide. The gel raft acts as a physical barrier to gastric reflux. The raft, which is located in the upper part of the stomach (fundus), also protects the oesophagus if reflux does occur as on reflux the raft contacts the oesophagus before the gastric juices, providing some further protection.

Examples of such products include Gaviscon® (Reckitt and Colman). This is described in GB 1524740 as a liquid or tablet formulation which forms an alginate raft to suppress gastric reflux. In practice, the raft forms relatively slowly. A similar alginate raft product Algicon® is described in WO 85/04806 (Rorer). This document is directed towards producing a composition of low sodium content. Other raft forming products currently marketed [e.g. Gastrocote® (Boeringer Mannheim), BiSoDol® (Whitehall) and Gastron® (Sanofi Winthrop)] also use alginates as the gel component.

Use of alginates as the gel forming component has various disadvantages. Alginates are extracted from various different brown seaweeds of the class Phaeophyceae which are found of the coast of a limited number of countries throughout the world. This leads to an uncertain source of supply. There is wide variation in the properties of the alginates isolated from different seaweeds due to the different ratio of mannuronic acid to guluronic acid monosaccharides in the alginate polysaccharide isolated from each species.
Polysaccharides which are particularly suitable for use in raft compositions are pectins. For a discussion and an outline of the nature of pectins, the reader is referred to Kirk-Othmer's Encyclopedia of Chemical Technology (1992) 4th Ed., Vol. 4: 943-944, published by John Wiley & Sons.

Pectins are naturally occurring polysaccharides located in the cell walls of all plant tissues which can be extracted from higher plant sources. Pectins comprise a linear chain of C₁₋₄ linked d-galactopyranosyluronic acid units, which makes the pectin molecule a d-galacturonan (a poly-d-galactopyranosyluronic acid) or a d-galacturonoglycan.

Pectins are divided according to the percentage of carbonyl groups esterified with methanol. Low methoxyl pectins (hereinafter referred to as LM pectins) have a degree of methoxylation (hereinafter referred to as DM) of less than 50 % and high methoxyl pectins (hereinafter referred to as HM pectins) have a DM of greater than 50 %. In addition, the degree of amidation (hereinafter referred as DA) indicates the percentage of carboxyl groups converted to the amide. In the presence of metal ions, such as calcium ions, pectin chains may cross-link more readily to form a more cohesive gel. The rigidity or strength of the gel and the amount of metal ion required to form the gel, are dependent on the DM and DA percentages for a particular pectin. HM pectins may form gels in the absence of metal ions due to hydrogen bonding between the pectin chains. LM pectins can be strengthened by the addition of calcium or other divalent or polyvalent metal ions.

Raft forming products comprising pectins as the gel forming ingredient are believed to be particularly suitable for relieving distress caused by gastric reflux for the following reasons. Pectin structure is largely independent of pH. The properties of pectins can be modified by selection of DM or DA values. Pectins break down in the higher pH of the lower intestine to their constituent galactopyranosyluronic acid monosaccharides which are easily absorbed. Pectins form a viscous liquid in the mouth which due to the good lipid binding properties of pectins will coat the lining of the oesophagus and stomach easily when swallowed, thus providing further protection against the effect of gastric reflux. Pectins are readily available from many common sources of supply such as discarded plant material like citrus peel or apple pomace.

EP 0286085 (Farma Foods) describes a solid dosage form antacid composition containing pectin as a raft forming component for treatment of gastric reflux. In order to obtain a stable long lasting gel in the stomach, this document teaches use of either a buffering agent or a pectin with a extremely low DM of less than 15 %. In addition the composition is directed towards long lasting neutralisation of the stomach contents in which the buffer and an antacid component are released from the gel gradually.

Scand. J. Gastroenterol., 1988, 23, p920-24, N Washington et al; describes a study which uses a pectin-casein mixture as the gelling agent in an anti-reflux formulation, comprising casein with 15 % pectin (w/w), 7 % potassium bicarbonate (w/w) and 19 % magnesium carbonate (w/w). The term 'w/w' as used herein indicates the percentage mass of a component per total mass of a composition. The casein acts as a buffering component to increase the lifetime of the gel by stabilising the pH in the immediate proximity of the gel. There is no teaching of the DM or DA values of the pectin used. The composition does not contain calcium ions.

Rev. Med. Chir. Soc. Med. Nat. lasi 1985 89 (2) p233-6 G Stanciu et al; describes a study comparing a sodium alginate composition with a pectin composition for treatment of gastric reflux. Both compositions are suspensions in which the alginate or pectin component are both described as a 'suspension agent having a neutralising effect'. The compositions further comprise an antacid mixture consisting of sodium bicarbonate, aluminium hydroxide and magnesia but no calcium. Both compositions are said to act by spreading as a thin film on the stomach acid contents'. The type of pectin used is not stated, but if a LM pectin was used a thick cohesive stable raft could not be formed in the absence of calcium ions to cross link the pectin chains. Thus the pectin composition disclosed could not form a stable, viscous, cohesive, foamed gel raft, and this paper teaches away from a raft forming product.

GB 2283171 (Reckitt & Colman) and GB 228372 (Scherer) are both co-pending British applications with the same priority date both published on 3 May 1995 which is after both of the two priority dates claimed for the present application. Both these publications are therefore of potential relevance only to the novelty of the present application and then only in some states. Both applications disclose raft forming chewable gelatine capsules in which the raft forming component may be inter alia a pectin in the presence of inter alia calcium. The DM of the pectin is not disclosed in either application and both applications disclose compositions which must comprise an oil or hydrophillic based vehicle.

Anti-reflux gel raft-forming agents act as a barrier to reflux or are refluxed in preference to the acidic gastric juices. These effects are entirely mechanical in nature and therefore antacids are not an essential component to prevent gastric reflux. Formation of a cohesive stable floating raft is dependent on the amount of acid present in the gastric environment both to react with the pectin to form a gel and to generate enough gas to foam the gel to lower its bulk density to enable it to float. Therefore too much antacid (more than about 30 % w/v) in a raft formulation may compete with the acid locally to such an extent that raft forming ability of the formulation is reduced and hence its effectiveness in protecting against gastric reflux is also impaired. In the presence of too much antacid the speed of raft formation may also be reduced which delays onset of relief of symptoms. The term 'w/v' as used herein indicates the percentage mass of a component (measured in grammes) per total volume of a composition (measured in millilitres).

Addition of a buffering component may also compete with the stomach acid and reduce the raft forming ability of the composition or the rapidity of raft formation. Buffering components are added to increase the stability of the raft over longer periods. Their disadvantage is that there is a danger that the rafts thus formed may be too stable, and would not break down easily in the higher pH found in the small intestine thus causing a blockage. As used herein a buffering component is defined to be a component which when present in solution in a suitable amount acts as a reservoir of or for hydrogen ions such that over a wide range of diluents the pH of the resultant solution is practically unchanged and which will resist a change of pH on addition of significant amounts of acid and/or alkali.

Therefore it would be advantageous if a composition could be produced using pectins as the raft forming ingredient which has a simple composition without a buffering component, which used pectins from readily available natural sources, and which rapidly formed a cohesive gel in the stomach, but which nevertheless would not block the gastrointestinal tract but would break up easily in the higher pH of the small intestine. The present invention aims to overcome the aforementioned and other problems of known compositions.

Therefore broadly in accordance with the present invention there is provided a composition in solid form which comprises as the sole gel forming agent, a pectin, or pharmaceutically acceptable salt thereof, which has a degree of methoxylation (DM) of from about 20 % to about 50 %; a calcium ion component; and one or more substances capable of producing a pharmaceutically acceptable gas at the pH normally physiologically present in the stomach, the composition forming a stable gel raft in a gastric environment; and the composition not containing a buffering component. Preferably the composition may does comprise a vehicle with an oil or hydrophillic base nor is it a gelatine capsule.

By "gastric environment" it is meant that the environment is one of acid pH, like the normal physiological pH of the stomach.

"Stable gel raft" as used herein means a cohesive gel structure which forms a physical barrier to gastric reflux.

The solid composition may be in any of the known solid dosage forms suitable for oral administration, such as cachets, caplets, capsules, dragées, extrudates, lozenges, pastilles, pellets, pills, tablets, troches and/or any mixtures thereof. The solid compositions may also be in the form of granules and/or powders which may be directly ingested by the patient or added to an acceptable fluid carrier (such as a suitable liquid e.g. water).

Preferably the pectin may be present in the composition in an amount from 1 % to 50 % by weight of the composition.

Preferably the degree of methoxylation (DM) of the pectin is from about 30 % to about 40 %, more preferably from about 35 % to about 40 %.

Preferably the degree of amidation (DA) of the pectin is from about 3% to about 23%.

Preferably the gas producing substance comprises one or more pharmaceutically acceptable salts selected from bicarbonate and carbonate and the pharmaceutically acceptable gas comprises carbon dioxide. More preferably, the gas producing substance is selected from potassium bicarbonate and calcium carbonate. The gas producing substance may be present in an amount from about 5 % to about 45 %, preferably from about 15 % to about 35 % and more preferably from about 25 % to about 35 %, by weight of the composition. Potassium bicarbonate is a favoured gas producing substance.

Advantageously the gas producing substance agent comprises two components, a first fast acting component (e.g. KHCO₃) to generate the initial bubbles which are trapped in the pectin gel whilst it is formed to create a foam, and a second slow acting component (e.g. CaCO₃) which produces gas over a longer period of time to refresh leakage of the gas entrapped in the gel.

The calcium ions aid cross linkage of the linear pectin chains to form a more cohesive gel which is stronger and forms a raft which is thicker than without the calcium ion component. The calcium ion component, which may be the same or different to the gas producing substance, may be present in an amount from about 1 % to about 25 % by weight of the composition. A preferred calcium ion component is calcium carbonate.

It would be further advantageous if the solid pectin raft forming compositions of the invention additionally comprised one or more further ingredients to aid patient compliance. Such further ingredients might comprise any of the following: agents which may help adjust, directly or indirectly, stomach pH to physiologically normal levels (for example antacids, proton pump inhibitors and/or H₂ receptor antagonists); anti-infective agents (for example antibiotics such as those effective against Helicobacter pylori either alone or as part of a combination therapy); agents which may act on neurotransmitters (for example anti-cholinergic agents which may block parasympathetically mediated secretory response and increased motility and/or local anaesthetics which may reduce pain and discomfort); anti-emetic agents which may act directly to eliminate or reduce stomach irritation, or indirectly to supress or reduce the reflux to vomit; and/or cytoprotectant agents which may act to enhance the secretion of protective mucus and/or bicarbonate, and/or provide a physical barrier to protect cells within the stomach and small intestine. Thus a preferred aspect of the present invention aims to provide compositions with such further ingredients, which have improved patient compliance over known compositions.

Therefore preferably compositions of the present invention further comprise one or more additional ingredients selected from: one or more antacids, one or more antibiotics, one or more anti-cholinergic agents, one or more anti-emetic agents, one or more cytoprotectants, one or more H₂ receptor antagonists, one or more local anaesthetics, one or more proton pump inhibitors and any suitable or compatible mixtures thereof. Compositions with such further ingredients may comprise a vehicle with an oil or hydrophillic base and/or a gelatine capsule.

Preferably the antacid may be present in an amount up to about 10% to about 30 % by weight of the composition. Preferably the antacid comprises aluminium magnesium hydroxy sulphate, more preferably of formula AlMg₁₀(OH)₃₁(SO₄)₂xH₂O (also known as magaldrate).

Preferably the antibiotic may be present in an amount up to about 1% to about 60%, more preferably about 10% to about 50 % by weight of the composition. Advantageously the antibiotic, which may comprise one or more components, (such as in triple or dual therapies) is effective against Helicobacter pylori. Preferably the antibiotic comprises: (2S,5R,6R)-6-[(R)-(-)-2-amino-2-(p-hydroxyphenyl)-acetamido]-3,3-dimethyl-7-oxo-4-thia-1-azabicyclo[3.2.0]heptane-2-carboxylic acid trihydrate (the INN [international non-propriety name] of which is amoxicilline and the BAN [British approved name] of which is amoxycillin).

Preferably the anti-cholinergic agent may be present in an amount from about 0.01% to about 30%, more preferably from about 0.5 to about 6% by weight of the composition. Preferably the anti-cholinergic agent comprises (bicyclohexyl)-1-carboxylic acid, 2-(diethylamino)ethyl ester hydrochloride (the INN and BAN of which is dicyclomine hydrochloride).

Preferably the anti-emetic agent may be present in an amount from about 0.5% to about 30% more preferably from about 0.5 to about 6% by weight of the composition. Preferably the anti-emetic agent comprises 4-amino-5-chloro-N-[2-(diethylamino) ethyl] -2-methoxybenzamide. (The BAN and INN of which is metoclopramide).

Preferably the cytoprotectant may be present in an amount from about 10% to about 50 % by weight of the composition. Preferably the cytoprotectant comprises tri-potassium di-citrato bismuthate.

Preferably the H₂ receptor antagonist may be present in an amount from about 0.5% to about 50%, more preferably from about 5% to about 30% by weight of the composition. Preferably the H₂ receptor antagonist comprises:N"-cyano-N-methyl-N'-[2-{5-methyl-1H-imidazol-4-yl)methyl}thio]-ethylguanidine (the INN and BAN of which is cimetidine); and/or N-*(*2-*{[(*-{-5-[(dimethylamino)methyl]-2-furanyl}methyl)thio*]*ethyl*})*-N-methyl-2-nitro-1,1-ethene-diamine (the INN and BAN of which is ranitidine).
Preferably the local anaesthetic may be present in an amount from about 0.5% to about 20%, more preferably from about 0.5% to about 3% by weight of the composition. Preferably the local anaesthetic comprises 2,2'-[(2-hydroxyethyl)-imino]bis[N-(1,1-dimethyl-2-phenylethyl)-N-methyl-acetamide (the INN of which is oxethazaine).

Preferably the proton pump inhibitor may be present in an amount from about 0.5% to 20%, more preferably from about 1% to about 6% by weight of the composition. Preferably the proton pump inhibitor comprises 5-methoxy-2{[4-methoxy-3,5-dimethyl-2-pyridyl)methyl]sulphinyl}benzimidazole (the INN and BAN of which is omeprazole).

The precise amount of the further ingredients specified herein which may be orally administered to a patient as part of the compositions of the present invention will depend on a number of factors, for example the severity of the condition, the patient being treated and the age and past medical history of the patient, and will lie within the sound discretion of any administering physician, pharmacist and/or other medical practitioner. For example a daily dose of the preferred further ingredients specified herein, (given in a single dose or in divided doses at one or more times during the day) which might be generally suitable for administration to adult human beings and generally therapeutically and/or prophylactically effective may comprise the following for each preferred ingredient: magaldrate from about 50 mg to about 500 mg; amoxycillin from about 50 mg to about 500 mg; dicyclomine hydrochloride from about 1 mg to about 100 mg; metoclopramide from about 1 mg to about 100 mg; tri-potassium di-citrato bismuthate from about 50 mg to about 500 mg; cimetidine from about 50 mg to about 500 mg; ranitidine from about 50 mg to about 500 mg; oxethazaine from about 1 mg to about 100 mg; and omeprazole from about 1 mg to about 100 mg.

The solid compositions of the present invention may be prepared by any suitable methods known to those skilled in the art. For example any pharmacologically active ingredients may be brought into association with suitable inert diluents, carriers and/or any other optional ingredients (for example those described herein). The ingredients in the compositions may be uniformly and/or intimately admixed and the resultant compositions may be shaped (for example by compressing and/or moulding). It will be appreciated by those skilled in the art that if the compositions of the present invention contain large amounts of excipients in relation to any active ingredients, repeated conventional mixing operations may be required to distribute the active ingredients evenly and/or homogenously throughout the composition. Compositions of the present invention may also be formulated in a manner known to those skilled in the art, to give a controlled release, for example rapid release or sustained release, of any active ingredients. Pharmaceutically acceptable diluents and/or carriers suitable for use in compositions of the present invention are well known in the art of pharmacy. The excipients used in the preparation of such compositions are the excipients known in the pharmacist's art.

The solid compositions of the present invention may comprise any known solid pharmaceutical forms suitable for oral administration such as cachets, caplets, capsules, dragées, extrudates, granules, lozenges, pastilles, pills, pellets, powders, tablets and/or troches.

The solid compositions of the present invention may be prepared by mixing any active ingredients with one or more of the following ingredients which are pharmaceutically acceptable: inert diluents, disintegrating agents, lubricants, binders and/or any mixtures thereof. It will be appreciated by those skilled in the art that a particular ingredient may perform more than one function (for example maize starch may act as a diluent, binder or disintegrating agent).

Inert diluents may comprise sugars (for example lactose, dextrin, sucrose, mannitol, powdered sugar and/or mixtures thereof), celluloses (for example microcrystalline cellulose), starches (for example maize starch, other pharmaceutical grade starch and/or mixtures thereof), clays (for example kaolin), calcium phosphate, calcium sulphate and/or mixtures thereof.

Disintegrating agents may comprise starches (for example maize starch, sodium starch glycolate and/or mixtures thereof), agar, bentonite, celluloses (for example methyl cellulose, carboxymethylcellulose, microcrystalline cellulose, hydroxypropyl cellulose and/or mixtures thereof), wood products, guar gum, croscarmellose sodium, sodium lauryl sulphate and/or mixtures thereof.

Lubricating agents may comprise magnesium stearate, calcium stearate, stearic acid, talc, paraffin sulphate, boric acid, sodium benzoate, sodium acetate, sodium chloride, leucine, polyethylene glycol and/or mixtures thereof.

Binders may comprise starches (for example maize starch), gelatin, sugars (for example sucrose, molasses, lactose and/or mixtures thereof) and/or natural and/or synthetic gums (for example acacia, sodium alginate, extract of Irish moss, celluloses [such as carboxymethylcellulose, hydroxypropylmethylcellulose, methylcellulose, ethylcellulose, micro-crystalline cellulose and/or mixtures thereof], polyethylene glycol, waxes, polyvinylpyrrolidone and/or mixtures thereof).

Solid compositions of the present invention may further comprise one or more of the following pharmaceutically acceptable ingredients and/or any suitable and/or compatible mixtures thereof: colouring agents; sweetening agents (for example intense sweeteners [such as aspartame and/or saccharin]); flavouring components (for example mints [such as peppermint]); preservatives (for example methyl p-hydroxybenzoate, propyl p-hydroxybenzoate and/or sorbic acid); anti-oxidants (for example vitamin C); wetting agents (for example sodium lauryl sulphate); and/or one or more pharmaceutically acceptable couples (for example those comprising an acid and a carbonate and/or bicarbonate salt) which effervesce to aid dissolution if the solid dosage form is added to water.

Solid compositions of the present invention may also optionally comprise one or more of other pharmaceutically acceptable ingredients and/or mixtures thereof, which are known in the art to permit production by known methods of solid forms for oral administration (for example blending, filling and/or tabletting). Such ingredients may comprise: agents to aid the flow of ingredients (for example talc and/or colloidal silicon dioxide); compression agents to increase the strength of the solid dosage form (for example sorbitol and/or lactose); and/or ionic and/or non-ionic surface active agents (for example sodium lauryl sulphate) to disperse any active ingredients within the solid composition and prevent grit forming at the surface of the solid composition.

Preferably the solid compositions are shaped to be more convenient for general use in oral administration.

The solid compositions may be formulated in a manner known to those skilled in the art so as to give a sustained release of any active ingredients. Enteric coated, solid forms comprising the composition may be advantageous, depending on the nature of any active ingredients. Various materials, for example shellac and/or sugar, may be present as coatings, or to otherwise modify the physical form of the solid forms. For example tablets and/or pills may, if desired, be provided with enteric coatings (such as membranes) by known methods, for example by the use of cellulose acetate phthalate, polyvinylacetate phthalate, hydroxypropylmethylcellulose phthalate and/or anionic polymers of methacrylic acid and/or its esters. To prevent and/or reduce cracking of the enteric coating during its application and/or storage of the solid composition, the enteric coating may comprise a plasticiser (for example diethylphthalate, tributylcitrate and/or triacetin).

Capsules and/or caplets (for example hard or soft gelatin capsules) comprising any active ingredients with or without added excipients may be prepared by conventional means and, if desired, provided with enteric coatings in a known manner. The contents of capsules and/or caplets may be formulated using known methods to give sustained release of any active ingredients.
The compositions of the present invention may be formulated into granules and/or powders with or without additional excipients. The granules and/or powders may be ingested directly by the patient or they may be added to suitable fluid carriers (for example a liquid such as water) before ingestion. The granules and/or powders may contain disintegrants (for example pharmaceutically acceptable effervescent couples formed from acids with carbonate and/or bicarbonate salts) to facilitate dispersion in fluid (for example liquid) media.

The invention will now be illustrated with reference to the following examples which are by of way of illustration only. All these examples were prepared in the same way. The examples comprise ingredients which are dry powders which were mixed together, the resultant dry powdered composition being compressed to form a tablet with a mass of approximately (1?) g. The tablets were tested by contact with 0.1M HCI solution to simulate a gastric environment. The tablets rapidly formed a stable gel raft on the surface of the liquid. At an alkali pH the gel raft disintegrated. Thus, these examples are particularly suitable for use in the prevention of gastric reflux. The amidated low methoxylated pectin used in all these examples was extracted from lemon peel (available commerically from Allied Colloids) and had a DM of 37% and a DA of 13%.

### Example 1

| | |
|---|---|
| Amidated low methoxylated pectin | 20% w/w |
| Potassium bicarbonate | 17% w/w |
| Calcium carbonate | 11% w/w |
| Sorbitol | 51% w/w |
| Magnesium stearate | 1% w/w |

### Example 2 - with an antacid

| | |
|---|---|
| Aluminium magnesium hydroxy sulphate | 20% w/w |
| Amidated low methoxylated pectin | 25% w/w |
| Potassium bicarbonate BPC | 20% w/w |
| Calcium carbonate BP | 11% w/w |
| Sorbitol | 23% w/w |
| Magnesium stearate | 1% w/w |

### Example 3 - with an H₂ receptor antagonist

| | |
|---|---|
| Cimetidine | 6.67% w/w |
| Amidated low methoxylated pectin | 20% w/w |
| Potassium bicarbonate | 17% w/w |
| Calcium carbonate | 11% w/w |
| Sorbitol | 44.33% w/w |
| Magnesium stearate | 1% w/w |

Examples 4 to 6 comprise a local anaesthetic:

### Example 4

| | |
|---|---|
| Amidated low methoxylated pectin | 20% w/w |
| Potassium bicarbonate | 17% w/w |
| Calcium carbonate | 11% w/w |
| Sorbitol | 46% w/w |
| Magnesium stearate | 1% w/w |
| Oxethazaine | 5% w/w |

### Example 5 - with an antacid

| | |
|---|---|
| Aluminium magnesium hydroxy sulphate | 20% w/w |
| Amidated low methoxylated pectin | 25% w/w |
| Potassium bicarbonate BPC | 20% w/w |
| Calcium carbonate BP | 11% w/w |
| Sorbitol | 18% w/w |
| Magnesium stearate | 1% w/w |
| Oxethazaine | 5% w/w |

### Example 6 - with an H₂ receptor antagonist

| | |
|---|---|
| Cimetidine | 6.67% w/w |
| Amidated low methoxylated pectin | 20% w/w |
| Potassium bicarbonate | 17% w/w |
| Calcium carbonate | 11% w/w |
| Sorbitol | 39.33% w/w |
| Magnesium stearate | 1% w/w |
| Oxethazaine | 5% w/w |

Examples 7 to 9 comprise an anti-cholinergic agent:

### Example 7

| | |
|---|---|
| Amidated low methoxylated pectin | 20% w/w |
| Potassium bicarbonate | 17% w/w |
| Calcium carbonate | 11 % w/w |
| Sorbitol | 46% w/w |
| Magnesium stearate | 1% w/w |
| Dicyclomine hydrochloride | 5% w/w |

### Example 8 - with an antacid

| | |
|---|---|
| Aluminium magnesium hydroxy sulphate | 20% w/w |
| Amidated low methoxylated pectin | 25% w/w |
| Potassium bicarbonate BPC | 20% w/w |
| Calcium carbonate BP | 11% w/w |
| Sorbitol | 23% w/w |
| Magnesium stearate | 1% w/w |
| Dicyclomine hydrochloride | 5% w/w |

### Example 9 - with an H₂ receptor antagonist

| | |
|---|---|
| Cimetidine | 6.67% w/w |
| Amidated low methoxylated pectin | 20% w/w |
| Potassium bicarbonate | 17% w/w |
| Calcium carbonate | 11% w/w |
| Sorbitol | 44.33% w/w |
| Magnesium stearate | 1% w/w |
| Dicyclomine hydrochloride | 5% w/w |

Examples 10 to 12 comprise an anti-emetic agent:

### Example 10

| | |
|---|---|
| Amidated low methoxylated pectin | 20% w/w |
| Potassium bicarbonate | 17% w/w |
| Calcium carbonate | 11% w/w |
| Sorbitol | 46% w/w |
| Magnesium stearate | 1% w/w |
| Metoclopramide | 5% w/w |

### Example 11 - with an antacid

| | |
|---|---|
| Aluminium magnesium hydroxy sulphate | 20% w/w |
| Amidated low methoxylated pectin | 25% w/w |
| Potassium bicarbonate BPC | 20% w/w |
| Calcium carbonate BP | 11% w/w |
| Sorbitol | 18% w/w |
| Magnesium stearate | 1% w/w |
| Metoclopramide | 5% w/w |

### Example 12 - with an H₂ receptor antagonist

| | |
|---|---|
| Cimetidine | 6.67% w/w |
| Amidated low methoxylated pectin | 20% w/w |
| Potassium bicarbonate | 17% w/w |
| Calcium carbonate | 11% w/w |
| Sorbitol | 44.33% w/w |
| Magnesium stearate | 1% w/w |
| Metoclopramide | 5% w/w |

## Claims

1. A composition in solid form which comprises as the sole gel raft-forming agent, a pectin or a pharmaceutically acceptable salt thereof, which has a degree of methoxylation of from about 20 % to about 50 %; a calcium ion component; and one or more substances capable of producing a pharmaceutically acceptable gas in the pH normally physiologically present in the stomach; the composition forming a stable gel raft in a gastric environment; and the composition not including a buffering component.

2. A composition as claimed in claim 1, in which the degree of methoxylation of the pectin is from about 30 % to about 40 %.

3. A composition as claimed in claim 1, in which the degree of amidation of the pectin is from about 3% to about 23%.

4. A composition as claimed in any preceding claim, in which the pectin is present in an amount from about 1 % to about 50 % by weight of the composition.

5. A composition as claimed in any preceding claim in which the calcium ion component is calcium carbonate.

6. A composition as claimed in any preceding claim, in which the calcium ion component is present in an amount of from about 1% to about 25% by weight of the composition.

7. A composition as claimed in any preceding claim, in which the gas producing substance comprises one or more pharmaceutically acceptable salts selected from bicarbonate and carbonate salts and the pharmaceutically acceptable gas comprises carbon dioxide.

8. A composition as claimed in any preceding claim, in which the gas producing substance is selected from potassium bicarbonate and calcium carbonate.

9. A composition as claimed in any preceding claim, in which the gas producing substance is present in an amount of from about 5% to about 45% by weight of the composition.

10. A composition as claimed in any preceding claim, further comprising one or more ingredients selected from: one or more antacids, one or more antibiotics, one or more anti-cholinergic agents, one or more anti-emetic agents, one or more cytoprotectants, one or more H₂ receptor antagonists, one or more local anaesthetics, one or more proton pump inhibitors and any suitable and compatible mixtures thereof.

11. A composition as claimed in claim 10, in which the further ingredient or ingredients, if present, are selected from: an antacid comprising magaldrate, an antibiotic comprising amoxycillin, an anti-cholingeric agent comprising dicyclomine hydrochloride, an anti-emetic agent comprising metochlopramide, a cytoprotectant comprising tri-potassium di-citrato bismuthate, a H₂ receptor antagonist comprising cimetidine and/or ranitidine, a local anaesthetic comprising oxethazaine, a proton pump inhibitor comprising omeprazole and any suitable and compatible mixtures thereof.

## Patentansprüche

1. Zubereitung in fester Form, die als ein einen Sol/Gel-Schwimmkörper (raft) bildendes Mittel ein Pektin oder ein pharmazeutisch annehmbares Salz desselben, das einen Methoxylierungsgrad von etwa 20 % bis etwa 50 % aufweist, eine Calcium-Ionen-komponente und eine oder mehrere Substanzen enthält, die bei dem normalerweise physiologisch im Magen vorliegenden pH ein pharmazeutisch annehmbares Gas erzeugen können, wobei die Zusammensetzung in einer gastrischen Umgebung einen stabilen Gel-Schwimmkörper (raft) bildet und wobei die Zubereitung keine Pufferkomponente enthält.

2. Zubereitung gemäß Anspruch 1, in der der Methoxylierungsgrad des Pektins von etwa 30 % bis etwa 40 % beträgt.

3. Zubereitung gemäß Anspruch 1, in der der Amidierungsgrad des Pektins von etwa 3 % bis etwa 23 % beträgt.

4. Zubereitung gemäß einem der vorstehenden Ansprüche, worin das Pektin in einer Menge von etwa 1 % bis etwa 50 % in Gewicht der Zubereitung vorliegt.

5. Zubereitung gemäß einem der vorstehenden Ansprüche, worin die Calcium-Ionenkomponente Calciumcarbonat ist.

6. Zubereitung gemäß einem der vorstehenden Ansprüche, worin die Calcium-Ionenkomponente in einer Menge von etwa 1 % bis etwa 25 % in Gewicht der Zubereitung vorliegt.

7. Zubereitung gemäß einem der vorstehenden Ansprüche, worin die Gas erzeugende Substanz ein oder mehrere pharmazeutisch annehmbare(s) Salz(e) umfasst, ausgewählt unter Bicarbonat-und Carbonatsalzen, und worin das pharmazeutisch annehmbare Gas Kohlendioxid umfasst.

8. Zubereitung gemäß einem der vorstehenden Ansprüche, worin die Gas erzeugende Substanz unter Kaliumbicarbonat und Calciumcarbonat ausgewählt ist.

9. Zubereitung gemäß einem der vorstehenden Ansprüche, worin die Gas erzeugende Substanz in einer Menge von etwa 5 % bis etwa 45 % in Gewicht der Zubereitung vorhanden ist.

10. Zubereitung gemäß einem der vorstehenden Ansprüche, zusätzlich umfassend einen oder mehrere Bestandteile ausgewählt unter: einem oder mehreren Antacida, einem oder mehreren Antibiotika, einem oder mehreren anticholinergen Mitteln, einem oder mehreren antiemetischen Mitteln, einem oder mehreren Zellschutzmitteln (cytoprotectants), einem oder mehreren H₂-Rezeptor-Antagonisten, einem oder mehreren Lokalanästhetika, einem oder mehreren Inhibitoren von Protonenpumpen und jeder geeigneten und verträglichen Mischung derselben.

11. Zubereitung gemäß Anspruch 10, worin der oder die weiteren Betandteile, falls vorhanden, ausgewählt sind unter: einem Magaldrat umfassenden Antacidum, einem Amoxycillin umfassenden Antibiotikum, einem Dicyclominhydrochlorid umfassenden anticholinergen Mittel, einem Metochlopramid umfassenden antiemetischen Mittel, einem Tri-kalium-di-citrato-bismuthat umfassenden Zellschutzmittel, einem Cimetidin und/oder Ranitidin umfassenden H₂-Rezeptor-Antagonisten, einem Oxethazain umfassenden Lokalanästhetikum, einem Omeprazol umfassenden Inhibitor einer Protonenpumpe und jede geeignete und verträgliche Mischung derselben.

## Revendications

1. Composition sous forme solide qui comprend, comme seul agent formant une nacelle de gel, une pectine ou un de ses sels pharmaceutiquement acceptables, qui a un degré de méthoxylation d'environ 20 % à environ 50 % ; un constituant renfermant des ions calcium ; et une ou plusieurs substances capables de produire un gaz pharmaceutiquement acceptable au pH normalement physiologiquement présent dans l'estomac ; la composition formant une nacelle de gel stable dans un environnement gastrique ; et la composition ne comprenant pas de tampon.

2. Composition suivant la revendication 1, dans laquelle le degré de méthoxylation de la pectine est compris dans l'intervalle d'environ 30 % à environ 40 %.

3. Composition suivant la revendication 1, dans laquelle le degré d'amidation de la pectine est compris dans l'intervalle d'environ 3 % à environ 23 %.

4. Composition suivant l'une quelconque des revendications précédentes, dans laquelle la pectine est présente en une quantité d'environ 1 % à environ 50 % en poids de la composition.

5. Composition suivant l'une quelconque des revendications précédentes, dans laquelle le constituant renfermant des ions calcium est le carbonate de calcium.

6. Composition suivant l'une quelconque des revendications précédentes, dans laquelle le constituant renfermant des ions calcium est présent en une quantité d'environ 1 % à environ 25 % en poids de la composition.

7. Composition suivant l'une quelconque des revendications précédentes, dans laquelle la substance productrice de gaz comprend un ou plusieurs sels pharmaceutiquement acceptables choisis entre des bicarbonates et carbonates et le gaz pharmaceutiquement acceptable comprend le dioxyde de carbone.

8. Composition suivant l'une quelconque des revendications précédentes, dans laquelle la substance productrice de gaz est choisie entre le bicarbonate de potassium et le carbonate de calcium.

9. Composition suivant l'une quelconque des revendications précédentes, dans laquelle la substance productrice de gaz est présente en une quantité d'environ 5 % à environ 45 % en poids de la composition.

10. Composition suivant l'une quelconque des revendications précédentes, comprenant en outre un ou plusieurs ingrédients choisis entre : un ou plusieurs antacides, un ou plusieurs antibiotiques, un ou plusieurs agents anti-cholinergiques, un ou plusieurs agents antiémétiques, un ou plusieurs cytoprotecteurs, un ou plusieurs antagonistes des récepteurs H₂, un ou plusieurs anesthésiques locaux, un ou plusieurs inhibiteurs de la pompe à protons et n'importe lequel de leurs mélanges convenables et compatibles.

11. Composition suivant la revendication 10, dans laquelle le ou les ingrédients supplémentaires, s'ils sont présents, sont choisis entre : un antacide comprenant le magaldrate, un antibiotique comprenant l'amoxycilline, un agent anti-cholinergique comprenant le chlorhydrate de dicyclomine, un agent anti-émétique comprenant le métochlopramide, un cytoprotecteur comprenant le dicitrato-bismuthate tripotassique, un antagoniste des récepteurs H₂ comprenant la cimétidine et/ou la ranitidine, un anesthésique local comprenant l'oxéthazaïne, un inhibiteur de la pompe à protons comprenant l'oméprazole et n'importe lequel de leurs mélanges convenables et compatibles.
